# FASCICULE DE BREVET EUROPEEN

(11) **EP 1 961 444 B1**
(45) Date de publication et mention de la délivrance du brevet: **16.11.2011**
(21) Numéro de dépôt: 08290178.6
(22) Date de dépôt: 26.02.2008
(51) Int. Cl.: A61N 1/05

(54) **Sonde non rectiligne et systéme pour neurostimulation électrique profonde comportant une telle sonde**
Nichtlineare Sonde und System zur tiefen Elektroneurostimulation, das eine solche Sonde umfasst
Non-rectilinear probe and system for deep brain stimulation including such a probe

(30) Priorité: 26.02.2007 FR 0701353
(43) Date de publication de la demande: 27.08.2008
(73) Titulaire: Commissariat à l'Énergie Atomique et aux Énergies Alternatives, 75015 Paris (FR)
(72) Inventeur: Benabid, Alim-Louis, 38240 Meylan (FR); Sauter-Starace, Fabien, 38170 Seyssinet-Pariset (FR); Caillat, Patrice, 38000 Grenoble (FR); Chabardes, Stephan, 38410 Saint Martin D'Uriage (FR)
(74) Mandataire: Priori, Enrico

(56) Documents cités:
- WO-A-02/078575
- US-A1- 2005 203 599
- US-A1- 2006 041 295
- US-B1- 7 033 326

## Description

L'invention porte sur une sonde pour électrostimulation cérébrale profonde, ainsi que sur un système pour électrostimulation cérébrale profonde comportant au moins une telle sonde.

L'électrostimulation cérébrale profonde (« deep brain stimulation » en langue anglaise) est une technique thérapeutique comportant l'implantation d'un appareil médical connu comme stimulateur cérébral, qui envoie des impulsions électriques à des parties spécifiques du cerveau. Par exemple, la stimulation des noyaux du thalamus ou du subthalamus peut être utilisée pour traiter des désordres moteurs tels que des tremblements, provoqués notamment par la maladie de Parkinson (voir l'article de A. - L Benabid., P. Pollak, C. Gervason, D. Hoffmann, D.-M. Gao, M. Hommel, J.-E. Perret et J. de Rougemont : « Long-term suppression of tremor by chronic stimulation of the ventral intermediate thalamic nucleus» The Lancet, Volume 337, N° 8738, 16 février 1991, Pages 403-406). En outre, la stimulation du cortex cingulaire subgenual est utilisée à titre expérimental pour le traitement de formes particulièrement graves et résistantes au traitement de dépression clinique (H.S. Mayberg et al. « Deep Brain Stimulation for Treatment-Resistant Depression », Neuron, Vol. 45, pages 651-660, 3 mars 2005). La stimulation est également testée au niveau des noyaux hypothalamiques postérieurs pour traiter les céphalées en grappe, de la matière grise périaqueducale pour atténuer la douleur et de l'hypothalamus ventromédian pour soigner certains cas d'obésité (A.-L. Benabid, B. Wallace, J. Mitrofanis, C. Xia, B. Piallat, V. Fraix, A. Batir, P. Krack, P. Pollak et F. Berger, « Therapeutic electrical stimulation of the central nervous system » Comptes Rendus Biologies, Volume 328, N° 2, février 2005, pages 177-186).

Dans tous les cas, une intervention d'électrostimulation cérébrale profonde comporte l'insertion dans le crâne du patient d'une sonde souple, guidée par un stylet rigide, jusqu'à ce que la pointe de ladite sonde atteigne la région du cerveau à stimuler. A son extrémité, la sonde comporte des électrodes ou contacts (généralement au nombre de quatre), qui sont reliées par un câble sous-cutané à un générateur d'impulsions, implanté à son tour sous la peau du patient comme un stimulateur cardiaque conventionnel. Après l'introduction de la sonde et sa fixation au crâne du patient, le stylet (ou mandrin) qui avait servi à la rigidifier est retiré de celle-ci, qui peut rester en place dans le cerveau du malade pendant une durée qui peut atteindre plusieurs années.
Une description plus détaillée de la procédure d'implantation d'une sonde pour électrostimulation cérébrale profonde est fournie par le document « Medtronic - DBS™ Lead Kit for Deep Brain Stimulation 3387 3389 - Implant manual » de la société Medtronic Inc., téléchargeable du site Internet :
http://www.medtronic.com/physician/activa/downloadablefiles/197928_b_006. pdf. ou dans de nombreuses publications scientifiques.

Des sondes pour électrostimulation cérébrale profonde de type conventionnel sont décrites, par exemple, dans le document précité de la société Medtronic, ainsi que dans le document US 6,512,958.

La forme rectiligne de la sonde est généralement imposée par la nécessité de rendre son insertion la moins traumatique possible pour le patient. Cependant, compte tenu des contraintes qui limitent le choix des trajets d'insertion pouvant être suivis par la sonde sans provoquer des lésions inacceptables du tissu cérébral, une telle forme ne permet pas toujours d'accéder facilement à des régions présentant un intérêt thérapeutique.

En outre, comme une sonde pour électrostimulation cérébrale profonde est implantée à demeure pour une durée de plusieurs années, il existe un risque important que sa pointe puisse se déplacer légèrement dans le temps, en compromettant son efficacité thérapeutique.

Le document EP1062973 décrit une sonde réalisée en matériau souple et présentant une extrémité de tête, ou distale, ayant une forme cintrée. La sonde est introduite dans le cerveau d'un patient à l'aide d'une canule rigide ; pendant qu'elle se trouve à l'intérieur de la canule, ladite extrémité de tête en épouse la forme rectiligne, pour recouvrir sa configuration cintrée lorsqu'elle en sort à la fin de l'opération d'insertion. Une telle sonde est principalement destinée à effectuer des mesures électrophysiologiques préalables à l'implantation d'une sonde d'électrostimulation proprement dite, la forme cintrée de son extrémité de tête permettant d'explorer plusieurs zones du cerveau sans avoir à l'extraire et à la réinsérer plusieurs fois. Par contre, elle convient moins bien à une implantation à demeure, car il est difficile d'extraire uniquement la canule du crâne du patient tout en laissant la sonde en place. En outre l'utilisation d'une canule d'insertion, dont le diamètre externe est nécessairement plus grand que celui de la sonde elle-même, rend l'intervention d'implantation plus traumatique.

Le document US-B-7, 033, 326 décrit le préambule.

Un but de l'invention est de remédier au moins à certains des inconvénients de l'art antérieur exposés ci-dessus.

Ce but est atteint par une sonde pour électrostimulation cérébrale profonde comportant : un corps tubulaire en matériau biocompatible ayant une paroi latérale définissant une lumière, ledit corps tubulaire pouvant être introduit pour au moins une partie de sa longueur à l'intérieur du corps d'un patient pour atteindre une région à stimuler ; une pluralité d'électrodes disposées à proximité d'une extrémité dite distale dudit corps tubulaire et pouvant être amenées en contact avec ladite région à stimuler à l'intérieur du corps dudit patient ; et un stylet rigide destiné à être introduit de manière amovible à l'intérieur de la lumière dudit corps tubulaire ; dans laquelle : ledit corps tubulaire a une forme d'équilibre non rectiligne, différente de celle du stylet et présentant un et un seul coude, et est suffisamment souple et élastique pour suivre la conformation dudit stylet en se déformant de manière réversible lorsque ce dernier est introduit dans sa lumière.

Ledit stylet peut présenter une forme rectiligne.

Le coude qui caractérise la sonde de l'invention permet d'atteindre des régions à stimuler qui ne seraient pas facilement accessibles par une sonde de type connu de l'art antérieur, à l'intérieur de cavités où l'extrémité de la sonde peut reprendre sa forme initiale sans léser des structures tissulaires. Ce coude peut être franc ou progressif, former un angle inférieur à 90° et de préférence compris entre 10° et 50° et présenter un rayon de courbure compris entre 1 mm et 5 cm environ.

De plus, le corps tubulaire d'une sonde selon l'invention peut présenter, à proximité de son extrémité distale, une région qui, à l'équilibre, a une forme de ballonnet, et qui est conformée de manière à s'allonger et à prendre une configuration sensiblement linéaire lors de l'introduction dudit stylet dans sa lumière. De cette manière la sonde peut être introduite, dans sa configuration linéaire, à l'intérieur d'une cavité du corps d'un patient, puis elle peut être bloquée à l'intérieur de ladite cavité en extrayant le stylet de manière à lui permettre de retrouver sa forme d'équilibre en ballonnet. Le ballonnet, étant situé au niveau de l'extrémité distale - c'est à dire de la pointe - de la sonde a également une fonction de sécurité, pour éviter que ladite pointe puisse léser le parenchyme du cerveau.

Avantageusement, des électrodes peuvent être prévues au niveau de ladite région en forme de ballonnet, afin de permettre la stimulation d'une région plus étendue du cerveau du patient.

En correspondance de la région en forme de ballonnet, la paroi latérale de la sonde peut présenter une épaisseur moindre que dans le restant dudit corps tubulaire. En variante ou en complément, en correspondance de ladite région en forme de ballonnet, ladite paroi latérale peut former des quartiers non jointifs, espacés par des fentes longitudinales susceptibles de se refermer suite à l'allongement provoqué par l'introduction dudit stylet dans la lumière.

Dans une sonde selon l'invention, ledit corps tubulaire peut être fermé en correspondance de son extrémité distale.

Le corps tubulaire peut être réalisé en un matériau choisi parmi les silicones, les siloxanes, les polyuréthanes, les polyvinyles chlorures, les benzocyclobutènes (BCB), les polyimides et les parylènes, ou tout autre matériau synthétique souple et biocompatible.

La longueur à l'équilibre de la sonde peut être comprise entre 15 cm et 40 cm.

Ledit corps tubulaire peut présenter une section sensiblement circulaire, avec un diamètre compris entre 0,5 et 5 mm, et de préférence entre 0,5 et 2 mm. Dans ce cas, le corps tubulaire de la sonde est fabriqué de préférence par moulage.

En variante, ledit corps tubulaire peut présenter une section sensiblement rectangulaire, avec une épaisseur comprise entre 10µm et 200µm et une largeur comprise entre 50µm et 3mm. Dans ce cas, le corps tubulaire de la sonde est fabriqué de préférence par dépôt de couches minces. La forme de l'extrémité de la sonde peut-être adaptée à la morphologie spécifique des structures neurales que l'on stimule.

Une sonde selon l'invention peut comprendre au moins un élément électriquement conducteur s'étendant dans la lumière ou à l'intérieur de la paroi latérale dudit corps tubulaire et formant des contacts électriques avec lesdites électrodes. Ledit ou chaque élément conducteur peut s'étendre jusqu'à une extrémité dite proximale, opposée à ladite extrémité distale, dudit corps tubulaire et comprendre des moyens de raccordement à un générateur d'impulsions électriques pour neurostimulation électrique profonde.

Inversement, cette sonde peut-être utilisée pour enregistrer des activités électriques générées à proximité des électrodes de contact.

Un autre objet de l'invention est un système de neurostimulation électrique profonde comportant : un générateur d'impulsions électriques pour neurostimulation électrique profonde ; et au moins une sonde telle que décrite ci-dessus, dont les électrodes sont raccordées électriquement audit générateur d'impulsions électriques.

La sonde de l'invention convient particulièrement au traitement :
- de la dépression chronique et des troubles obsessionnels compulsifs par stimulation du noyau accumbens ou de toute zone périventriculaire ;
- de la douleur par stimulation de la matière grise périaqueducale ;
- des migraines en grappe par stimulation des noyaux hypothalamiques postérieurs ;
- de certains désordres alimentaires par stimulation de l'hypothalamus ventromédian ;
- des troubles de la mémoire par stimulation des structures limbiques proches de la paroi ventriculaire, comme les tubercules mamillaires, le trigone et ses piliers, et les faisceaux mamillo-thalamiques ; ou
- de tout autre trouble fonctionnel qui répondrait à la stimulation de structures neurales périventriculaires.

D'autres caractéristiques, détails et avantages de l'invention ressortiront à la lecture de la description faite en référence aux dessins annexés donnés à titre d'exemple et qui représentent, respectivement :
- La figure 1, une vue en coupe longitudinale d'une sonde selon l'invention dans sa configuration d'insertion, avec un stylet rectiligne rigide introduit dans la lumière de son corps tubulaire ;
- Les figures 2, 3 et 4, des vues en coupe longitudinale de sondes selon un premier, un deuxième et un troisième modes de réalisation de l'invention, respectivement, dans leur configuration d'équilibre, sans stylet ;
- La figure 5, une vue frontale depuis l'extrémité distale d'une sonde selon un quatrième mode de réalisation de l'invention, également dans sa configuration d'équilibre, sans stylet ;
- Les figures 6, 7, 8 et 9, des exemples d'applications de sondes selon différents modes de réalisation de l'invention ;
- La figure 10, une vue en coupe transversale selon la ligne X-X d'une sonde selon l'invention, présentant une section sensiblement circulaire ;
- La figure 11, un procédé de fabrication d'une sonde selon le premier ou deuxième mode de réalisation de l'invention et présentant une section sensiblement circulaire;
- La figure 12, un procédé de fabrication d'une sonde selon le troisième ou quatrième mode de réalisation de l'invention et présentant une section sensiblement circulaire;
- La figure 13, une vue en coupe transversale selon la ligne X-X d'une sonde selon l'invention, présentant une section sensiblement rectangulaire ; et
- La figure 14, un schéma de principe d'un système d'électrostimulation cérébrale profonde comprenant une sonde selon ledit premier mode de réalisation de l'invention, introduite dans le troisième ventricule du cerveau d'un patient.

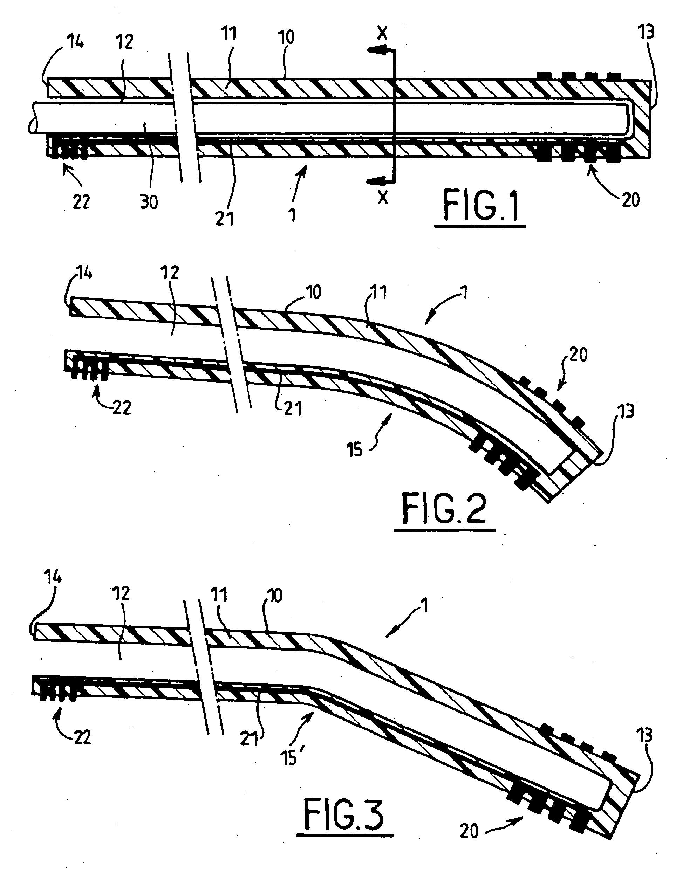

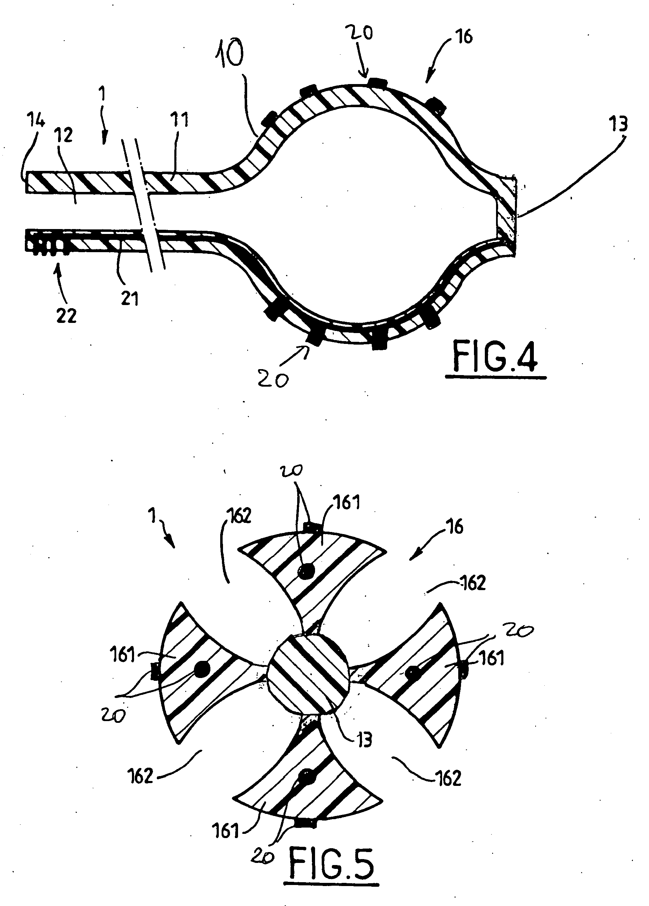

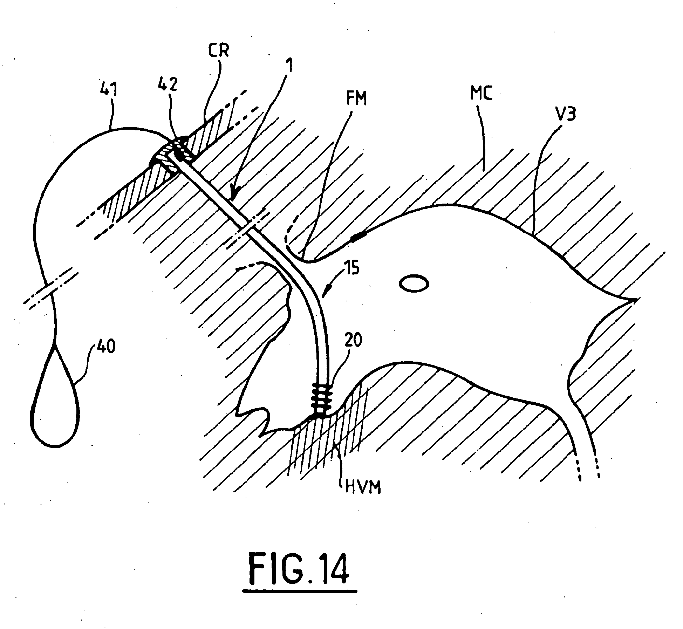

Comme cela est représenté sur les figures 1 à 5, une sonde 1 pour neurostimulation électrique profonde selon l'invention comporte un corps tubulaire 10 ayant une paroi latérale 11 qui définit une lumière 12, et un stylet rigide 30 pouvant être introduit dans ladite lumière 12. L'insertion du stylet 30 est rendue possible par le fait qu'une extrémité 14, dite proximale, du corps tubulaire 10 est ouverte; l'extrémité opposée 13, dite distale, est généralement fermée de manière à empêcher la sortie dudit stylet, et permettre par son intermédiaire d'exercer une pression pour rendre rectiligne l'électrode. Des électrodes 20, généralement au nombre de quatre, sont disposés à proximité de ladite extrémité distale 13 du corps tubulaire 10 et dépassent de sa paroi latérale 11 de manière à pouvoir être amenés en contact avec une région à stimuler du corps d'un patient suite à l'insertion de la sonde 1 dans ledit corps. Les électrodes 20 peuvent avoir une forme annulaire, entourant le corps tubulaire 10, comme cela est visible sur la figure 5, mais cela n'est pas essentiel et dépend de l'application envisagée. Les électrodes 20 sont reliées à des moyens de raccordement 22, situés à proximité de l'extrémité proximale 12, par l'intermédiaire d'éléments conducteurs 21, tels que des fils ou des pistes métalliques, s'étendant dans la lumière 12 du corps tubulaire 10 ou à l'intérieur de sa paroi latérale 11. Les moyens de raccordement 22 permettent la connexion de la sonde 1 à un générateur d'impulsions électriques pour neurostimulation cérébrale (référence 40 sur la figure 14).

La longueur de la sonde est généralement comprise entre 15 et 40 cm, la valeur exacte étant fonction de l'application spécifique envisagée.

Comme représenté sur les figures 2 à 4, la forme d'équilibre du corps tubulaire 10 n'est pas rectiligne ; par forme d'équilibre on entend la forme que le corps tubulaire 10 prend lorsqu'il n'est soumis à aucune force externe et lorsque le stylet 30 est extrait de sa lumière interne 12. Par exemple, selon un premier mode de réalisation représenté sur la figure 2, la portion distale 15 dudit corps tubulaire 10 présente un coude progressif sur une longueur comprise entre 15 et 20mm. Selon un deuxième mode de réalisation (figure 3), le corps tubulaire 10 présente un coude franc 15', situé près de son extrémité distale fermée 13, à environ 10mm de cette dernière.

On entend par « coude franc » 15' une variation brusque de direction entre deux segments sensiblement rectilignes juxtaposés, et par «coude progressif » 15 une région arrondie réalisant une variation progressive de direction. En général, le rayon de courbure du coude sera compris entre 1 mm (coude franc) et 5 cm (coude très progressif).

Selon un troisième et un quatrième mode de réalisation (figures 4 et 5), ledit corps tubulaire 10 présente, en plus du coude 15 ou 15', un élargissement définissant une région 16 en forme de ballonnet. Cette région présente une longueur d'environ 10 mm, et est généralement située à proximité de l'extrémité distale de la sonde. Le diamètre du ballonnet en configuration ouverte est de préférence compris entre 2 et 8mm. Le diamètre et la forme du ballonnet (sphérique, ovoïde ou multiforme, ..) peut varier en fonction de la cavité à l'intérieur de laquelle la sonde sera introduite.

Quel que soit le mode de réalisation considéré, le matériau dans lequel est réalisé le corps tubulaire 10 de la sonde 1 doit être suffisamment souple et élastique pour suivre la conformation du stylet 30 en se déformant de manière réversible lorsque ce dernier est introduit dans la lumière 12. Ainsi, l'insertion du stylet 30 provoque le redressement de la sonde. Dans le cas du troisième et du quatrième mode de réalisation, en outre, la pointe du stylet 30 prend appui sur l'extrémité distale fermée 13 du corps tubulaire 10 et provoque ainsi son allongement ; l'allongement se produit de manière préférentielle en correspondance de la région 16 en forme de ballonnet, de telle manière que la sonde prend une configuration sensiblement linéaire.

Dans ce troisième mode de réalisation (figure 4), la paroi latérale 11 peut présenter une épaisseur réduite en correspondance de sa région 16 en forme de ballonnet, de manière à en obtenir l'allongement préférentiel précité lors de l'insertion du stylet 30.

Le quatrième mode de réalisation de l'invention (figure 5) se caractérise par le fait que, dans la région 16 en forme de ballonnet, sa paroi latérale 11 n'est pas continue, mais est constituée d'une pluralité de quartiers 161 non jointifs, espacés par des fentes longitudinales 162. Lorsque le stylet 30 est introduit dans la lumière 12 et prend appui sur l'extrémité distale 13 de la sonde, la force de traction qui s'exerce sur ladite paroi latérale 11 tends à redresser les quartiers 161 et à fermer les fentes 162. La paroi latérale peut également présenter une épaisseur réduite en correspondance de la région 16 en forme de ballonnet, mais cela n'est pas essentiel, car l'allongement préférentiel est assuré par la présence des fentes 162.

Le ballonnet 16 a une triple fonction. Premièrement, il permet le blocage de la sonde à l'intérieur d'un ventricule du cerveau. Deuxièmement il évite que la pointe de la sonde puisse, par un mouvement involontaire, pénétrer dans le parenchyme du cerveau du patient et le léser. Troisièmement, des électrodes 20 peuvent être disposées au niveau du ballonnet, afin de permettre la stimulation d'une région cérébrale plus étendue que dans le cas du premier et du deuxième mode de réalisation, la forme d'équilibre pouvant dans ce cas être préformée afin de s'adapter à la forme de la cavité dans laquelle elle se développe.

L'utilisation d'un ballonnet du type représenté sur les figures 4 et 5 peut s'avérer avantageuse également dans le cas d'une sonde rectiligne, ne présentant pas de coude.

Etant destiné à être introduit dans le corps d'un patient, et plus particulièrement dans son cerveau, le corps tubulaire 10 doit être réalisé, en totalité ou au moins en ce qui concerne sa partie externe, en matériau biocompatible. Des matériaux biocompatibles présentant des propriétés de souplesse et d'élasticité suffisantes pour la mise en oeuvre de l'invention sont, par exemple, les silicones, les siloxanes, les polyuréthanes, les polyvinyles chlorures, les benzocyclobutènes (BCB), les polyimides et les parylènes. L'épaisseur de la paroi latérale 11 du corps tubulaire 10 doit également être choisie de manière à assurer la souplesse et l'élasticité recherchées, mais également une résistance mécanique adéquate. Par exemple, une épaisseur comprise entre 100µm et 1 mm peut convenir pour la plupart des applications.

Les électrodes 20 doivent également être réalisées en métal biocompatible, par exemple en platine iridié (Pt-Ir 90-10). Les autres éléments conducteurs, qui ne sont pas destinés à entrer en contact avec le corps d'un patient mais qui seront *a priori* choisis biocompatibles, peuvent également être réalisés en graphite, ITO (oxyde d'indium et étain), ou des alliages à haute limite élastique type MP35N, etc.

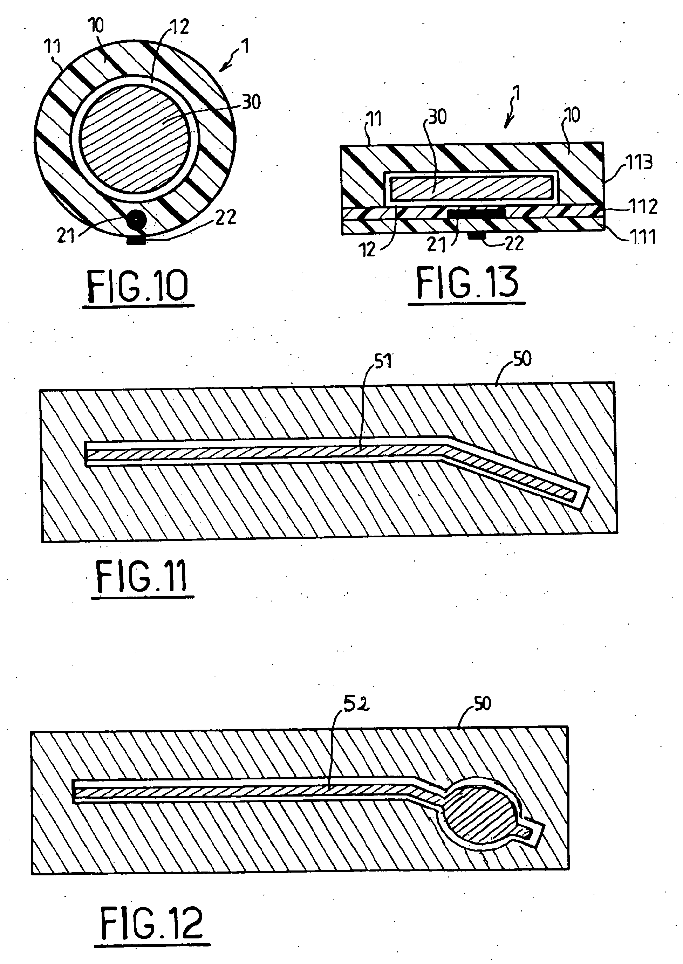

La figure 10 montre une coupe transversale d'une sonde 1 dont le corps tubulaire 10 présente une section sensiblement circulaire, et donc une forme générale cylindrique, avec une extrémité distale 13 arrondie (sphérique). Le diamètre du corps tubulaire est généralement compris entre 0,5 et 5 mm, et de préférence entre 0,5 et 2 mm, la valeur exacte dépendant de l'application spécifique considérée. Une sonde à section transverse circulaire peut être obtenue par une technique conventionnelle de moulage par injection, comme représenté sur les figures 11 et 12. Un insert 51, 52 introduit dans le moule 50 est utilisé pour réaliser la lumière intérieure 12 ; les électrodes 20, les éléments conducteurs internes 21 et les moyens de raccordement 22 sont également introduits dans le moule 50 avant l'injection de la matière plastique ou élastomère constituant la paroi latérale 11. Dans le cas d'une sonde du type à ballonnet, l'insert 52 ne peut pas être extrait simplement du corps tubulaire 10 après la fabrication de ce dernier. Pour résoudre ce problème, il est possible d'utiliser un insert 52 en cire ou en paraffine, pour pouvoir l'extraire à l'état fondu, à l'instar du procédé de moulage à cire perdue.

Des procédés de moulage permettent également la fabrication de sondes ayant une section non circulaire mais, par exemple, elliptique ou polygonale.

En variante une sonde de forme sensiblement rectangulaire, telle que représentée sur la figure 13, peut être fabriquée par dépôt de couches minces par des procédés tels que le dépôt par centrifugation, les niveaux conducteurs (électrodes 20, conducteurs internes 21, moyens de raccordement 22) étant réalisés par des techniques empruntées aux technologies des semi-conducteurs ou des circuits imprimés. La lumière 12 peut être obtenue à l'aide d'une couche sacrificielle. Une sonde de ce type peut présenter typiquement une largeur comprise entre 50µm et 3mm et une épaisseur comprise entre 10 µm et 200 µm.

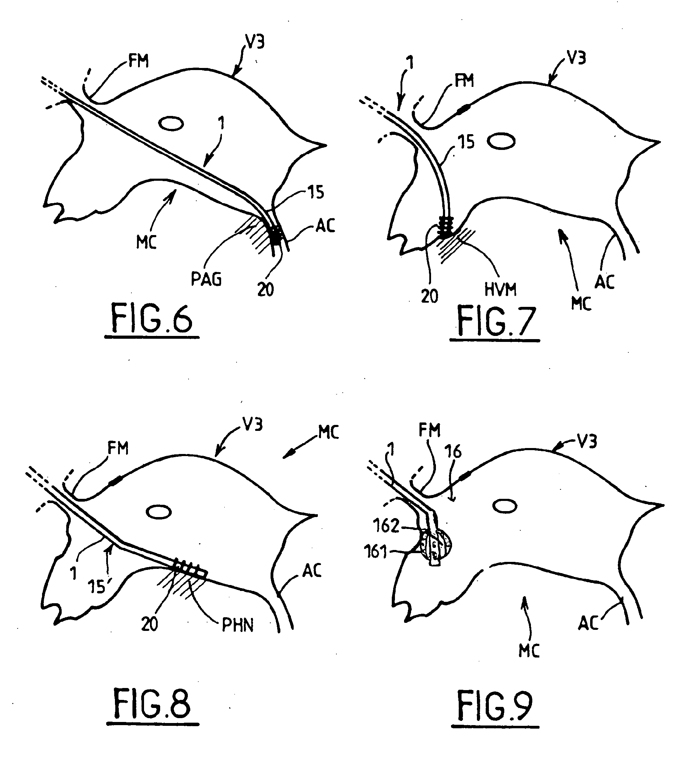

Les figures 6 à 9 montrent des exemples d'applications de sondes selon différents modes de réalisation de l'invention. Dans la plupart des cas, la sonde est introduite dans le troisième ventricule V3 d'un cerveau humain, qui est rempli de liquide céphalorachidien, qui n'oppose aucune résistance au retour du corps tubulaire 10 à sa forme d'équilibre après l'extraction du stylet 30. Dans l'exemple de la figure 6, une sonde courbe pénètre dans l'aqueduc AC pour atteindre la substance grise périaqueducale PAG afin de traiter la douleur. Dans l'exemple de la figure 7, une sonde du même type est utilisée pour atteindre l'hypothalamus ventromédian HVM afin de traiter certains troubles alimentaires responsables de cas d'obésité. Dans l'exemple de la figure 8, une sonde coudée stimule les noyaux hypothalamiques postérieurs afin de traiter les céphalées en grappe. Dans tous ces cas, la sonde 1 est introduite dans le troisième ventricule V3 en passant par le foramen de Monro FM, afin de minimiser les lésions provoquées à la matière cérébrale MC. On comprend que l'utilisation d'une sonde conventionnelle de forme rectiligne ne permettrait pas d'atteindre ces cibles thérapeutiques tout en suivant une trajectoire optimale du point de vue des dommages collatéraux.

Dans l'exemple de la figure 9, une sonde du type de la figure 5 est introduite dans le troisième ventricule et bloquée à cet endroit par l'ouverture du ballonnet. Bien que cela ne soit pas clairement visible sur la figure, au moins deux quartiers 161 du ballonnet 16 rentrent en contact avec le parenchyme du cerveau du patient : on obtient ainsi une meilleure stimulation que dans le cas d'une sonde « linéaire ».

Bien entendu, il ne s'agit là que d'exemples non limitatifs d'applications possibles de l'invention.

Dans tous les cas considérés ici, il a été supposé que le stylet 30 présente une forme rectiligne. En réalité, il ne s'agit nullement d'une caractéristique essentielle de l'invention : au contraire, l'utilisation d'un stylet courbe, mais de forme différente de celle du corps tubulaire 10 à l'équilibre, peut s'avérer avantageuse afin de minimiser les dommages collatéraux provoqués par l'introduction de la sonde 1 dans le corps du patient, et faciliter la progression de la sonde dans la cavité pour pouvoir s'y orienter.

La figure 14 montre de manière schématique un système d'électrostimulation cérébrale utilisant une sonde 1 courbe, représentée à l'état déployé à l'intérieur du troisième ventricule V3 du cerveau d'un patient, avec ses électrodes 20 en contact avec l'hypothalamus ventromédian HVM. D'une manière connue en soi, le corps tubulaire 10 s'étend à travers la matière cérébrale MC jusqu'à ressortir du crâne du patient par l'intermédiaire d'un orifice pratiqué dans la boîte crânienne CR de ce dernier. Au niveau de cet orifice, le corps tubulaire est solidarisé à la voûte selon les moyens habituels de cette chirurgie (ligature, bouchon 42, clip, ciment, ...). En correspondance de l'extrémité postérieure du corps tubulaire 10, les éléments conducteurs 21 sont reliés par l'intermédiaire de moyens de raccordement 22 à un câble sous-cutané 41 qui est à son tour relié à un générateur d'impulsions électriques pour neurostimulation profonde 40. Le raccordement entre la sonde 1 et le câble sous-cutané 41 peut être réalisé grâce à un connecteur placé sous l'épicrâne (périoste crânien) à distance de l'orifice de sortie de l'électrode.

## Revendications

1. Sonde (1) pour électrostimulation cérébrale profonde comportant :
- un corps tubulaire (10) en matériau biocompatible ayant une paroi latérale (11) définissant une lumière (12), ledit corps tubulaire (10) pouvant être introduit pour au moins une partie de sa longueur à l'intérieur du corps d'un patient pour atteindre une région à stimuler (PAG, HVM, PHN) ;
- une pluralité d'électrodes (20) portées par ledit corps tubulaire et pouvant être amenées en contact avec ladite région à stimuler (PAG, HVM, PHN) à l'intérieur du corps dudit patient ; et
- un stylet rigide (30) destiné à être introduit de manière amovible à l'intérieur de la lumière (12) dudit corps tubulaire (10) par une extrémité dite proximale de ce dernier; dans lequel :
- ledit corps tubulaire (10) présente une forme d'équilibre non rectiligne, différente de celle du stylet (30), comportant un seul coude (15, 15') qui sépare deux parties dudit corps tubulaire, dites partie proximale et partie distale, non alignées l'une par rapport à l'autre ; et est suffisamment souple et élastique pour suivre la conformation dudit stylet (30) en se déformant de manière réversible lorsque ce dernier est introduit dans sa lumière (12) ;
**caractérisé en ce que:**
- lesdites électrodes (20) sont portées par ladite partie distale du corps tubulaire (10), à proximité d'une extrémité dite distale de ce dernier, opposée à ladite extrémité proximale.

2. Sonde (1) selon la revendication 1, dans laquelle ledit stylet (30) présente une forme rectiligne.

3. Sonde (1) selon l'une des revendications précédentes, dans laquelle ledit coude est un coude progressif (15).

4. Sonde (1) selon l'une des revendications 1 ou 2, dans laquelle ledit coude est un coude franc (15').

5. Sonde (1) selon l'une des revendications précédentes, dans laquelle ledit coude (15, 15') forme un angle inférieur à 90° et de préférence compris entre 10° et 50°.

6. Sonde (1) selon l'une des revendications précédentes, dans laquelle ledit coude (15, 15') présente un rayon de courbure compris entre 1 mm et 5 cm.

7. Sonde (1) selon l'une des revendications précédentes, dans laquelle ledit corps tubulaire (10) présente, à proximité de son extrémité distale, une région qui, à l'équilibre, a une forme de ballonnet (16), et qui est conformée de manière à s'allonger et à prendre une configuration sensiblement linéaire lors de l'introduction dudit stylet (30) dans sa lumière (12).

8. Sonde (1) selon la revendication 7 dans laquelle des électrodes (20) sont prévues au niveau de ladite région en forme de ballonnet (16).

9. Sonde (1) selon la revendication 7 ou 8 dans laquelle, en correspondance de ladite région en forme de ballonnet (16), ladite paroi latérale (11) présente une épaisseur moindre que dans le restant dudit corps tubulaire (10).

10. Sonde (1) selon l'une des revendications 7 à 9 dans laquelle, en correspondance de ladite région en forme de ballonnet (16), ladite paroi latérale (11) forme des quartiers non jointifs (161), espacés par des fentes longitudinales (162) susceptibles de se refermer suite à l'allongement provoqué par l'introduction dudit stylet (30) dans la lumière (12).

11. Sonde (1) selon l'une des revendications précédentes, dans laquelle ledit corps tubulaire (10) est fermé en correspondance de son extrémité distale (13).

12. Sonde (1) selon l'une des revendications précédentes, dans laquelle ledit corps tubulaire (10) est réalisé en un matériau choisi parmi les silicones, les siloxanes, les polyuréthanes, les polyvinyles chlorures, les benzocyclobutènes (BCB), les polyimides et les parylènes.

13. Sonde (1) selon l'une des revendications précédentes, présentant une longueur à l'équilibre comprise entre 15 cm et 40 cm.

14. Sonde (1) selon l'une des revendications précédentes, dans laquelle ledit corps tubulaire (10) présente une section sensiblement circulaire, avec un diamètre compris entre 0,5 et 5 mm, et de préférence entre 0,5 et 2mm.

15. Sonde (1) selon l'une des revendications 1 à 12, dans laquelle ledit corps tubulaire (10) présente une section sensiblement rectangulaire, avec une épaisseur comprise entre 10 μm et 200 μm et une largeur comprise entre 50 μm et 3 mm.

16. Sonde (1) selon l'une des revendications précédentes, comprenant au moins un élément électriquement conducteur (21) s'étendant dans la lumière (12) ou à l'intérieur de la paroi latérale (11) dudit corps tubulaire (10) et formant des contacts électriques avec lesdites électrodes (20).

17. Sonde (1) selon la revendication 16, dans laquelle ledit ou chaque élément conducteur (21) s'étend jusqu'à une extrémité dite proximale (14), opposée à ladite extrémité distale (13), dudit corps tubulaire (10) et comprend des moyens (22) de raccordement à un générateur d'impulsions électriques pour neurostimulation électrique profonde (40).

18. Système pour électrostimulation cérébrale profonde comportant :
- un générateur d'impulsions électriques pour neurostimulation électrique profonde (40) ; et
- au moins une sonde (1) selon l'une des revendications précédentes, dont les électrodes (20) sont raccordées électriquement audit générateur d'impulsions électriques (40).

## Claims

1. A lead (1) for deep brain electrical stimulation, the lead comprising:
a tubular body (10) of biocompatible material having a side wall (11) defining a lumen (12), said tubular body (10) being suitable for being inserted over at least a fraction of its length into the inside of a patient's body in order to reach a region for stimulation (PAG, HVM, PHN);
a plurality of electrodes (20) carried by said tubular body and capable of being brought into contact with said region for stimulation (PAG, HVM, PHN) within the body of said patient; and
a rigid stylet (30) for inserting removably into the lumen (12) of said tubular body (10) through a so-called "proximal" end thereof; wherein
staid tubular body (10) has an equilibrium shape that is not rectilinear, being different from the shape of the stylet (30), and presenting one and only one bend (15, 15') separating two parts of said tubular body, called "proximal" and "distal" parts, which are not aligned with each other; and is sufficiently flexible and elastic to follow the shape of said stylet (30) by deforming reversibly when the stylet is inserted into the lumen (12);
**characterized in that:**
s aid electrodes (20) are carried by said "distal" part of the tubular body (10), near a so-called "distal" end thereof, opposite to said "proximal" end.

2. A lead (1) according to claim 1, in which said stylet (30) is rectilinear in shape.

3. A lead (1) according to either preceding claim, in which said bend is a progressive bend (15).

4. A lead (1) according to claim 1 or claim 2, in which said bend is a sharp bend (15').

5. A lead (1) according to any preceding claim, in which said bend (15, 15') forms an angle of less than 90°, and preferably lying in the range 10° to 50°.

6. A lead (1) according to any preceding claim, in which said bend (15, 15') presents a radius of curvature lying in the range 1 mm to 5 cm.

7. A lead (1) according to any preceding claim, in which said tubular body (10) presents, close to its distal end, a region that, when at equilibrium, forms a balloon (16), and that is shaped in such a manner as to lengthen and take on a configuration that is substantially linear when said stylet (30) is inserted into the lumen (12).

8. A lead (1) according to claim 7, in which electrodes (20) are provided at said balloon-shaped region (16).

9. A lead (1) according to claim 7 or claim 8, in which, in correspondence with said balloon-shaped region (16), said side wall (11) presents thickness that is smaller than in the remainder of said tubular body (10).

10. A lead (1) according to any one of claims 7 to 9, in which, in correspondence with said balloon-shaped region (16), said side wall (11) forms non-touching quarters (161) that are spaced apart by longitudinal slots (162) capable of closing up as a result of the elongation caused by inserting said stylet (30) into said lumen (12).

11. A lead (1) according to any preceding claim, in which said tubular body (10) is closed in correspondence with its distal end (13).

12. A lead (1) according to any preceding claim, in which the said tubular body (10) is made of a material selected from silicones; siloxanes; polyurethanes; polyvinyl chlorides; benzocyclobutenes (BCBs); polyimides; and parylenes.

13. A lead (1) according to any preceding claim, presenting a length at equilibrium lying in the range 15 cm to 40 cm.

14. A lead (1) according to any preceding claim, in which said tubular body (10) presents a section that is substantially circular, having a diameter lying in the range 0.5 mm to 5 mm, and preferably in the range 0.5 mm to 2 mm.

15. A lead (1) according to any one of claims 1 to 12, in which said tubular body (10) presents a section that is substantially rectangular, with thickness lying in the range 10 μm to 200 μm and width lying in the range 50 μm to 3 mm.

16. A lead (1) according to any preceding claim, including at least one electrically conductive element (21) extending in the lumen (12) or within the side wall (11) of said tubular body (10) and forming electrical contacts with said electrodes (20).

17. A lead (1) according to claim 16, in which said or each conductive element (21) extends to a "proximal" end (14) of said tubular body (10), opposite from its said distal end (13), and includes connection means (22) for connection to an electrical pulse generator for deep electrical neurostimulation (40).

18. A system for deep brain electrical stimulation, the system comprising:
an electrical pulse generator for deep electrical neurostimulation (40); and
at least one lead (1) according to any preceding claim, in which the electrodes (20) are electrically connected to said electrical pulse generator (40).

## Patentansprüche

1. Sonde (1) zur tiefen zerebralen Elektrostimulation, umfassend:
- einen röhrenförmigen Körper (10) aus einem biokompatiblen Material mit einer seitlichen Wand (11), welche ein Lumen (12) definiert, wobei der röhrenförmige Körper (10) für mindestens einen Teil seiner Länge in das Innere des Körpers eines Patienten eingeführt werden kann, um eine zu stimulierende Region (PAG, HVM, PHN)zu erreichen;
- eine Vielzahl von Elektroden (20), welche von dem röhrenförmigen Körper getragen sind und mit der zu stimulierenden Region (PAG, HVM, PHN) im Inneren des Körpers des Patienten in Kontakt gebracht werden können; und
- ein starres Stilett (30), welches dazu bestimmt ist, auf herausnehmbare Weise in das Innere des Lumens (12) des röhrenförmigen Körpers (10) durch ein proximal genanntes Ende des letzteren eingeführt zu werden; wobei:
- der röhrenförmige Körper (10) eine nicht geradlinige Gleichgewichtsform aufweist, welche sich von derjenigen des Stiletts (30) unterscheidet, welche eine einzige Biegung (15, 15')aufweist, die zwei Teile des röhrenförmigen Körpers, genannt proximaler Teil und distaler Teil, welche nicht miteinander fluchten, voneinander trennt; und hinreichend weich und elastisch ist, um der Formgebung des Stiletts (30) zu folgen und sich auf reversible Weise zu verformen, wenn das letztere in sein Lumen (12) eingeführt wird;
**dadurch gekennzeichnet, dass:**
- die Elektroden (20) von dem distalen Teil des röhrenförmigen Körpers (10) getragen sind, in der Nähe eines distal genannten Endes des letzteren, welches entgegengesetzt zu dem proximalen Ende ist.

2. Sonde (1) nach Anspruch (1), wobei das Stilett (30) eine geradlinige Form aufweist.

3. Sonde (1) nach einem der vorhergehenden Ansprüche, wobei die Biegung eine progressive Biegung (15) ist.

4. Sonde (1) nach einem der Ansprüche 1 oder 2, wobei die Biegung eine scharfe Biegung (15') ist.

5. Sonde (1) nach einem der vorhergehenden Ansprüche, wobei die Biegung (15, 15') einen Winkel bildet, welcher geringer als 90° ist und bevorzugt zwischen 10° und 50° enthalten ist.

6. Sonde (1) nach einem der vorhergehenden Ansprüche, wobei die Biegung (15, 15') einen Krümmungsradius aufweist, welcher zwischen 1 mm und 5 cm enthalten ist.

7. Sonde (1) nach einem der vorhergehenden Ansprüche, wobei der röhrenförmige Körper (10) in der Nähe seines distalen Endes eine Region aufweist, welche im Gleichgewicht eine Ballonform (16) aufweist, und welche derart geformt ist, dass sie sich bei Einführung des Stiletts (30) in ihr Lumen (12) verlängert und eine im Wesentlichen lineare Konfiguration einnimmt.

8. Sonde (1) nach Anspruch 7, wobei die Elektroden (20) auf Höhe der Region mit Ballonform (16) vorgesehen sind.

9. Sonde (1) nach Anspruch 7 und 8, wobei in Entsprechung zu der Region mit Ballonform (16) die seitliche Wand (11) eine Dicke aufweist, welche kleiner ist als bei dem Rest des röhrenförmigen Körpers (10).

10. Sonde (1) nach einem der Ansprüche 7-9, wobei in Entsprechung zu der Region mit Ballonform (16) die seitliche Wand (11) nicht aneinander angrenzende Teile (161) bildet, welche durch longitudinale Schlitze (162) voneinander beabstandet sind, welche dazu geeignet sind, sich infolge der durch das Einführen des Stiletts (30) in das Lumen (12) hervorgerufenen Verlängerung zu verschließen.

11. Sonde (1) nach einem der vorhergehenden Ansprüche, wobei der röhrenförmige Körper (10) in Entsprechung zu seinem distalen Ende (13) verschlossen ist.

12. Sonde (1) nach einem der vorhergehenden Ansprüche, wobei der röhrenförmige Körper (10) aus einem Material realisiert ist, welches ausgewählt ist aus Silikonen, Siloxanen, Polyurethanen, Polyvinylchloriden, Benzocyclobutenen (BCB), Polyimiden und Parylenen.

13. Sonde (1) nach einem der vorhergehenden Ansprüche, welche im Gleichgewicht eine Länge aufweist, welche zwischen 15 cm und 40 cm enthalten ist.

14. Sonde (1) nach einem der vorhergehenden Ansprüche, wobei der röhrenförmige Körper (10) einen im Wesentlichen kreisförmigen Querschnitt aufweist, mit einem Durchmesser, welcher zwischen 0,5 und 5 mm und bevorzugt zwischen 0,5 und 2 mm enthalten ist.

15. Sonde (1) nach einem der Ansprüche 1-12, wobei der röhrenförmige Körper (1) einen im Wesentlichen rechtwinkligen Querschnitt aufweist, mit einer Dicke, welche zwischen 10 µm und 200 µm enthalten ist, und einer Breite, welche zwischen 50 µm und 3 mm enthalten ist.

16. Sonde (1) nach einem der vorhergehenden Ansprüche, umfassend wenigstens ein elektrisch leitfähiges Element (21), welches sich in dem Lumen (12) oder im Inneren der seitlichen Wand (11) des röhrenförmigen Körpers (10) erstreckt und elektrische Kontakte mit den Elektroden (20) bildet.

17. Sonde (1) nach Anspruch 16, wobei das oder jedes leitfähige Element (21) sich bis zu einem proximal genannten Ende (14), entgegengesetzt zu dem distalen Ende (13), des röhrenförmigen Körpers (10) erstreckt und Mittel (22) zum Anschluss an einen Generator für elektrische Impulse zur tiefen elektrischen Neurostimulation (40) aufweist.

18. System zur tiefen zerebralen Elektrostimulation, umfassend:
- einen Generator für elektrische Impulse zur tiefen elektrischen Neurostimulation (40);
- wenigstens eine Sonde (1) nach einem der vorhergehenden Ansprüche, deren Elektroden (20) elektrisch an den Generator für elektrische Impulse (40) angeschlossen sind.
